# EUROPEAN PATENT APPLICATION

(11) **EP 4 268 803 A1**
(43) Date of publication of application: **01.11.2023**
(21) Application number: 21909391.1
(22) Date of filing: 21.12.2021
(51) Int. Cl.: A61K 9/08, A61K 39/395, A61K 47/12, A61P 35/00, A61P 37/02

(54) **STABLE ANTIBODY PREPARATION, PREPARATION METHOD FOR SAME, AND APPLICATIONS THEREOF**

(30) Priority: 22.12.2020 WO PCT/CN2020/138273
(71) Applicant: Bio-Thera Solutions, Ltd., Guangzhou, Guangdong 510530 (CN)
(72) Inventor: YANG, Yili, Guangzhou, Guangdong 510530 (CN); LUO, Xiaoping, Guangzhou, Guangdong 510530 (CN); LI, Qingrui, Guangzhou, Guangdong 510530 (CN); KOU, Zixuan, Guangzhou, Guangdong 510530 (CN); WU, Yong, Guangzhou, Guangdong 510530 (CN); LIU, Cuihua, Guangzhou, Guangdong 510530 (CN)
(74) Representative: Engelhard, Markus
(86) International application number: PCT/CN2021/140083
(87) International publication number: WO 2022/135395

(57) **Abstract**

Related to the field of bioformulations, provided is a stable antibody formulation, comprising an anti-IL-17 antibody, a buffer, a stabilizer, and a surfactant, where the stabilizer is selected from methionine or a combination of methionine and a sugar alcohol, and the pH value of the antibody formulation is 5.4-6.6. The antibody formulation has improved stability at high temperature and room temperature, can be kept in a liquid form, and is convenient to use.

## Description

### TECHNICAL FIELD

The present invention belongs to the field of biological formulations, and relates to a stable antibody formulation.

### BACKGROUND

An anti-IL-17 antibody has significant efficacy in treating various autoimmune diseases, such as rheumatoid arthritis, psoriatic arthritis, diabetes, asthma, chronic plaque psoriasis, multiple sclerosis, and the like. As a protein product, the anti-IL-17 antibody easily degrades and has poor stability, so it needs to be prepared into a stable formulation to ensure its effect. At present, there are a variety of anti-IL-17 antibody formulations available, but there is still a need in the art for stable formulations of anti-IL-17 antibodies.

### SUMMARY

The present invention is intended to provide a stable anti-IL-17 antibody formulation so as to solve the problems in the prior art.

In a first aspect, the present invention provides a stable antibody formulation comprising an anti-IL-17 antibody, a buffer, a stabilizer, and a surfactant, wherein the stabilizer is selected from methionine and a combination of methionine and a sugar alcohol.

In one embodiment, the methionine is at a concentration of 0.15%-1%. In one embodiment, the methionine is at a concentration of 0.4% or 1%. In one embodiment, the sugar alcohol is at a concentration of 3%-5%. In one embodiment, the sugar alcohol is at a concentration of 3% or 4%.

In one embodiment, the antibody formulation has a pH value of 5.4-6.6. In one embodiment, the antibody formulation has a pH value of 5.8-6.6. In one embodiment, the antibody formulation has a pH value of 5.8-6.4. In one embodiment, the antibody formulation has a pH value of 6.1.

In one embodiment, the buffer is a citrate buffer or a phosphate buffer. In one embodiment, the buffer is at a concentration of 10-30 mM. In one embodiment, the buffer is 20 mM citrate buffer. In one embodiment, the buffer is 20 mM phosphate buffer. In one embodiment, the buffer is a combination of 10 mM phosphate buffer and 10 mM citrate buffer.

In one embodiment, the stabilizer is a combination of methionine and sorbitol. In one embodiment, the methionine is at a concentration of 0.15%-1%. In one embodiment, the methionine is at a concentration of 1% or 0.4%. In one embodiment, the sorbitol is at a concentration of 3%-5%. In one embodiment, the sorbitol is at a concentration of 3% or 4%.

In one embodiment, the surfactant is Tween 80 or Tween 20. In one embodiment, the surfactant is at a concentration of 0.01%-0.04%. In one embodiment, the surfactant is at a concentration of 0.02%. In one embodiment, the surfactant is Tween 80. In one embodiment, the Tween 80 is at a concentration of 0.02%.

In one embodiment, the anti-IL-17 antibody is at a concentration of 8-300 mg/mL. In one embodiment, the anti-IL-17 antibody is at a concentration of 20-300 mg/mL. In one embodiment, the anti-IL-17 antibody is at a concentration of 100-300 mg/mL. In one embodiment, the anti-IL-17 antibody is at a concentration of 150 mg/mL.

In one embodiment, the anti-IL-17 antibody has a light chain sequence set forth in SEQ ID NO: 1, and a heavy chain sequence set forth in SEQ ID NO: 2. In one embodiment, the anti-IL-17 antibody is a monoclonal antibody of IgG1/kappa subtype. In one embodiment, the anti-IL-17 antibody is expressed by a CHO cell.

In one embodiment, the formulation of the present invention comprises 150 mg/mL anti-IL-17 antibody, 20 mM citrate buffer, 3% sugar alcohol, 0.4% methionine, and 0.02% Tween 80, with a pH value of 5.8-6.4. In some embodiments, the pH value is 6.1.

In one embodiment, the formulation of the present invention comprises 150 mg/mL anti-IL-17 antibody, 20 mM citrate buffer, 3% sorbitol, 0.4% methionine, and 0.02% Tween 80, with a pH value of 5.8-6.4. In some embodiments, the pH value is 6.1.

In one embodiment, the formulation of the present invention does not comprise a sugar.

In a second aspect, the present invention provides a method for preparing the formulation, comprising: taking a specified amount of an anti-IL-17 antibody, a buffer, a stabilizer, and a surfactant, adding water for dissolving, and mixing the above substances uniformly. In some embodiments, the present invention provides a method for preparing the formulation, comprising: taking a specified amount of an anti-IL-17 antibody, citric acid monohydrate, sorbitol, methionine, and Tween 80, adding water for dissolving, mixing the above substances uniformly, adjusting the volume to a specified volume, and adjusting the pH value to 5.8-6.6, preferably 5.8-6.4 with NaOH. In some embodiments, the present invention provides a method for preparing the formulation, comprising: taking a specified amount of an anti-IL-17 antibody, citric acid monohydrate, sodium citrate dihydrate, sorbitol, methionine, and Tween 80, adding water for dissolving, mixing the above substances uniformly, and adjusting the volume to a specified volume.

In a third aspect, the present invention provides use of the formulation in the preparation of a product for treating an autoimmune disease including, but not limited to, rheumatoid arthritis, psoriatic arthritis, diabetes, asthma, chronic plaque psoriasis, and multiple sclerosis.

In a fourth aspect, the present invention provides an antibody pharmaceutical product for treating an IL-17-associated disease comprising the anti-IL-17 antibody formulation described above and a container for storing the formulation. The pharmaceutical product may also comprise instructions for use. The container may be any container conventionally used in the art for storing drugs, such as pre-filled containers, pre-filled syringes, vials, ampoules, sachets, and the like. The antibody formulation of the present invention has improved stability both at high temperature and at room temperature, can be kept in a liquid form, and is convenient to use.

### BRIEF DESCRIPTION OF THE DRAWINGS

FIG. 1 shows the trend of change in SEC-HPLC monomer purity of the antibody in different buffers over time under the high-temperature condition;
FIG. 2 shows the trend of change in SEC-HPLC aggregates of the antibody in different buffers over time under the high-temperature condition;
FIG. 3 shows the trend of change in IEC-HPLC main peak of the antibody in different buffers over time under the high-temperature condition;
FIG. 4 shows the trend of change in CE-SDS (NR) main peak of the antibody in different buffers over time under the high-temperature condition;
FIG. 5 shows the trend of change in SEC-HPLC monomer content of the antibody in different buffers over time under the light condition;
FIG. 6 shows the trend of change in SEC-HPLC aggregates content of the antibody in different buffers over time under the light condition;
FIG. 7 shows the trend of change in IEC-HPLC main peak of the antibody in different buffers over time under the light condition; and
FIG. 8 shows the trend of change in CE-SDS (NR) main peak content of the antibody in different buffers over time under the light condition.

### DETAILED DESCRIPTION

The present invention will be illustrated by the following specific embodiments, but the content of the present invention is not limited thereto.

It should be noted that "%" related to components of the liquid formulation in the present invention refers to weight/volume (w/v) percent, wherein the weight unit may be g, and the volume unit may be mL. For example, 1% stabilizer in a solution means 1 g stabilizer in 100 mL solution, or the stabilizer content is 0.01 g/mL.

"About" refers to a general error range for corresponding values as readily understood by those skilled in the relevant art. In some embodiments, "about" mentioned herein refers to the values described and ranges thereof of ±10%, ±5%, or ±1%.

"Comprise" or "include" means that the compositions, methods, and the like comprise the listed elements (e.g., components of the compositions, steps of the methods, and the like), but do not exclude the others. When used to define the compositions and methods, "consisting essentially of..." means excluding other elements that have a fundamental impact on the combination for its intended use, but not excluding elements that do not substantially affect the characteristics of the compositions or methods. "Consisting of..." means excluding elements not specifically listed. Embodiments defined by each of these transition terms are all within the scope of the present invention. For example, when a composition is described as including components A, B, and C, a composition consisting essentially of A, B, and C, and a composition consisting of A, B, and C are independently within the scope of the present invention.

The term "buffer", also known as a buffer system, includes, but is not limited to, organic acid salts, such as salts of succinic acid, acetic acid, citric acid, ascorbic acid, gluconic acid, carbonic acid, tartaric acid, or phthalic acid; Tris, or inorganic acid salts, such as phosphate buffers. In addition, amino acid components may be also used as buffers. Such amino acid components include, but are not limited to, glycine, histidine, arginine, lysine, ornithine, isoleucine, leucine, alanine, glutamic acid, or aspartic acid. In some embodiments, the buffer is a histidine salt buffer. The amount of the buffer in the present invention means the total amount of the buffer pair in the buffer system constituting the buffer. In some embodiments, molar concentration is taken as a unit of the amount of the buffer, the value of which refers to the molar concentration of the buffer pair in the buffer system of the buffer. For example, where a citrate buffer consisting of citric acid and sodium citrate is used as the buffer, a given concentration of the citrate buffer (e.g., 20 mM) is the combined concentration of citric acid and sodium citrate (e.g., 2.8 mM citric acid, 17.2 mM sodium citrate, etc.).

The formulation of the present invention can be prepared with the excipients or the hydrate thereof. For example, citric acid can be anhydrous citric acid, or citric acid hydrate, such as citric acid monohydrate; for example, 0.59 mg of citric acid monohydrate includes 0.59 mg of citric acid monohydrate or a corresponding amount of citric acid.

The term "sugar alcohol" includes, but is not limited to, mannitol, xylitol, maltitol, lactitol, sorbitol (also known as sorbit or glucitol), and the like.

The term "surfactant" includes, but is not limited to, Tween (e.g., Tween 20 and Tween 80); poloxamers (e.g., poloxamer 188); Triton; sodium dodecyl sulfate (SDS); sodium lauryl sulfate; sodium octyl glycoside; lauryl sulfobetaine, myristyl sulfobetaine, linoleyl sulfobetaine, or stearyl sulfobetaine; lauryl sarcosine, myristyl sarcosine, linoleyl sarcosine, or stearyl sarcosine; linoleyl betaine, myristyl betaine, or cetyl betaine; lauramidopropyl betaine, cocamidopropyl betaine, linoleamidopropyl betaine, myristamidopropyl betaine, palmitamidopropyl betaine, or isostearamidopropyl betaine (e.g., lauramidopropyl); myristamidopropyl dimethylamine, palmitamidopropyl dimethylamine, or isostearamidopropyl dimethylamine; sodium methyl cocoyl taurate or disodium methyl oleyl taurate; polyethylene glycol, polypropylene glycol, and copolymers of ethylene and propylene glycol (e.g., Pluronics, PF68, etc.); and the like. In some embodiments, the surfactant is Tween 80.

Tween is also known as polysorbate (e.g., Tween 20 is known as polysorbate 20, and Tween 80 is known as polysorbate 80).

The "treatment" of a patient's disease means: (1) preventing a disease from developing in a patient who is prone to the disease or has not shown symptoms of the disease; (2) inhibiting the disease or symptoms of the disease, or blocking the progression of the disease; or (3) alleviating or eliminating the disease or symptoms of the disease, or making it regress.

"Stability" and "stable" herein mean that the antibody (including the antibody fragment thereof) does not, or only minimally, undergo aggregation, degradation, or fragmentation under given conditions of manufacture, preparation, transportation and/or storage in a liquid formulation comprising the antibody. A "stable" formulation maintains biological activity under given conditions of manufacture, preparation, transportation and/or storage. The stability of the antibody can be evaluated by the extent of aggregation, degradation, or fragmentation of the formulation and the like as measured by technologies such as SEC-HPLC, IEC-HPLC, CE-SDS (NR), visual inspection and turbidity, sub-visible particles, and detection of particle size by DLS. The active ingredient in the formulation of the present invention is an anti-IL-17 antibody, including but not limited to monoclonal antibodies, chimeric antibodies, dAbs (domain antibodies), single-chain antibodies, Fab, Fab- and F(ab')2 fragments, Fv, scFvs and Fab expression libraries. In some embodiments, the anti-IL-17 antibody is a recombinant anti-IL-17 monoclonal antibody, which is prepared by culturing cells that efficiently express the recombinant anti-IL-17 monoclonal antibody, isolating the antibody, and purifying the antibody to a high degree. In some embodiments, the light chain of the anti-IL-17 antibody has an amino acid sequence set forth in SEQ ID NO: 1, and the heavy chain has an amino acid sequence set forth in SEQ ID NO: 2. In some embodiments, the anti-IL-17 antibody is secukinumab, such as an antibody in COSENTYX^{®} and biosimilars thereof.

In some embodiments, the anti-IL-17 antibody has a content of about 100-300 mg/mL. In some embodiments, the anti-IL-17 antibody has a content of about 120-200 mg/mL. In some embodiments of the present invention, the anti-IL-17 antibody has a content of about 140-160 mg/mL, for example, about 100 mg/mL, about 120 mg/mL, about 140 mg/mL, about 145 mg/mL, about 150 mg/mL, about 155 mg/mL, about 160 mg/mL, about 180 mg/mL, about 200 mg/mL, about 250 mg/mL, about 300 mg/mL, or a range between any two of these values (inclusive) or any value therein. In some embodiments, the anti-IL-17 antibody has a content of about 150 mg/mL.

In some embodiments, the formulation provided herein has a pH of about 5.0-7.0. In some embodiments, the formulation provided herein has a pH of about 5.4-6.6. In some embodiments, the pH is about 5.8-6.6. In some embodiments, the pH is about 6.0-6.4, for example, about 5.0, about 6.0, about 6.1, about 6.2, about 6.3, about 6.4, about 6.5, about 6.6, about 7.0, or a range between any two of these values (inclusive) or any value therein. In some embodiments, the pH is about 6.1.

In some embodiments, the buffer of the present invention includes one or more of a citrate buffer, a phosphate buffer, a succinate buffer, and an acetate buffer. In some embodiments, the buffer is a citrate buffer. In some embodiments, the buffer is a combination of a phosphate buffer and a citrate buffer.

In some embodiments, the buffer is at a concentration in the range of about 10-30 mM. In some embodiments, the buffer is at a concentration of about 15-25 mM. In some embodiments, the buffer is at a concentration of about 18-22 mM, for example, about 10 mM, about 15 mM, about 18 mM, about 19 mM, about 20 mM, about 21 mM, about 22 mM, about 25 mM, about 30 mM, or a range between any two of these values (inclusive) or any value therein. In some embodiments, the buffer is 20 mM citrate buffer.

In some embodiments, the formulation further comprises a stabilizer. In some embodiments, the stabilizer is selected from one or more of sodium chloride, mannitol, sorbitol, sucrose, trehalose, arginine hydrochloride, and methionine. In some embodiments, the stabilizer is a combination of sorbitol and methionine.

In some embodiments, the stabilizer is at a concentration of about 0.1%-10%. In some embodiments, the stabilizer is at a concentration of about 0.2%-5%, for example, about 0.2%, about 0.4%, about 1%, about 2%, about 3%, about 4%, about 5%, or a range between any two of these values (inclusive) or any value therein. In some embodiments, the stabilizer is about 4% sorbitol. In some embodiments, the stabilizer is a combination of about 4% sorbitol and about 1% methionine. In some embodiments, the stabilizer is a combination of about 3% sorbitol and about 0.4% methionine.

In some embodiments, the formulation further comprises a surfactant. In some embodiments, the surfactant is Tween 80, Tween 20, or poloxamer 188. In some embodiments, the surfactant is Tween 80.

In some embodiments, the surfactant is at a concentration of about 0.01%-0.04%, or about 0.01%-0.03%, or about 0.015%-0.025%, or about 0.02%. In some embodiments, the surfactant is at a concentration of about 0.01%, about 0.015%, about 0.02%, about 0.025%, about 0.03%, about 0.04%, or a range between any two of these values (inclusive) or any value therein. In some embodiments, the surfactant is about 0.02% Tween 80.

In some embodiments, the formulation comprises the following components: about 100-300 mg/mL of an anti-IL-17 antibody, about 10-30 mM of a buffer, about 0.1%-10% of a stabilizer, and about 0.01%-0.04% of a surfactant, with a pH of about 5.0-7.0. In some embodiments, the antibody formulation comprises the following components: about 120-200 mg/mL of an anti-IL-17 antibody, about 15-25 mM of a buffer, 0.2%-5% of a stabilizer, and 0.01%-0.03% of a surfactant, with a pH of about 5.8-6.6. In some embodiments, the antibody formulation comprises the following components: about 140-160 mg/mL of an anti-IL-17 antibody, about 15-25 mM citrate buffer, about 3%-5% sorbitol and/or about 0.4%-1% methionine, and about 0.01%-0.03% Tween 80, with a pH of about 5.8-6.6. In some embodiments, the antibody formulation comprises the following components: about 140-160 mg/mL secukinumab, about 18-22 mM citrate buffer, about 3%-5% sorbitol and/or about 0.4%-1% methionine, and about 0.015%-0.025% Tween 80, with a pH of about 5.8-6.6. In some embodiments, the antibody formulation comprises the following components: about 150 mg/mL secukinumab, about 20 mM citrate buffer, about 4% sorbitol, and about 0.02% Tween 80, with a pH of about 5.8-6.6. In some embodiments, the antibody formulation comprises the following components: about 150 mg/mL secukinumab, about 20 mM citrate buffer, about 4% sorbitol, about 1% methionine, and about 0.02% Tween 80, with a pH of about 5.8-6.6. In some embodiments, the antibody formulation comprises the following components: about 150 mg/mL secukinumab, about 20 mM citrate buffer, about 3% sorbitol, about 0.4% methionine, and about 0.02% Tween 80, with a pH of about 5.8-6.6. In some embodiments, the antibody formulation comprises the following components: about 150 mg/mL secukinumab, about 0.59 mg/mL citric acid monohydrate, about 5.06 mg/mL sodium citrate dihydrate, about 30 mg/mL sorbitol, about 4 mg/mL methionine, and about 0.2 mg/mL Tween 80, with a pH of about 5.8-6.6.

In summary, the present invention provides a preferred liquid formulation combination scheme, wherein the buffer system of the formulation includes a citrate buffer, the stabilizer of the formulation includes sorbitol and methionine, and the surfactant of the formulation includes Tween 80 or Tween 20. The formulation has a pH value range of 5.0-7.0.

The present invention provides a liquid aqueous pharmaceutical formulation comprising antibodies suitable for therapeutic use, which is convenient to administer and contains high protein concentrations, primarily for the treatment of disorders caused by IL-17. In some embodiments, the pharmaceutical formulation has the effect of enhancing stability. In other embodiments, the formulation of the present invention is stable after at least 5 freeze-thaw cycles. In other embodiments, the formulation of the present invention remains stable after being placed at a high temperature of 40 °C for 1 week, 2 weeks, 3 weeks, or 4 weeks, for example, the IEC main peak remains above 65%. In other embodiments, the formulation of the present invention remains stable after being placed at 25 °C for 1 month, 2 months, or 3 months, for example, the IEC main peak remains above 65%. In other embodiments, the formulation of the present invention still remains stable under a light condition of 4000 lx for a period of time.

The present invention provides a formulation comprising an effective amount of the antibody component. In one embodiment of the present invention, the formulation is a formulation, for example, suitable for intravenous injection or subcutaneous injection. In one embodiment of the present invention, the antibody is at a concentration of about 150 mg/mL in the liquid aqueous pharmaceutical formulation.

The present invention further provides a method for treating an IL-17-associated disease, comprising administering to a patient the antibody formulation.

In some embodiments, the dosage of the antibody is about 10-500 mg/dose for the IL-17-associated disease. In some embodiments, the dosage of the antibody is about 100-350 mg/dose, or about 150-300 mg/dose for the IL-17-associated disease. In some embodiments, the dosage of the antibody is about 10 mg/dose, about 100 mg/dose, about 150 mg/dose, about 200 mg/dose, about 300 mg/dose, about 400 mg/dose, or about 500 mg/dose for the IL-17-associated disease. In some embodiments, the antibody formulation is administered to a patient at a frequency of about once every 1 week to every 6 weeks. In some embodiments, the antibody formulation is administered to a patient at a frequency of once every 1 week, and once every 4 weeks after 4 weeks. In some embodiments, the antibody formulation is administered to a patient at a dosage of about 150 mg/dose once every 1 week, and once every 4 weeks after 4 weeks. In some embodiments, the antibody formulation is administered to a patient at a dosage of about 300 mg/dose once every 1 week, and once every 4 weeks after 4 weeks.

In the following examples, reagents and instruments used are those conventional in the art and commercially available unless otherwise specified; the methods used are conventional in the art, and those skilled in the art can undoubtedly implement the methods and obtain the corresponding results according to the content of the examples.

The information on some devices is as follows:
peristaltic pump manufacturer: Longer Pump, model: BT300-1F
ultrafiltration membrane manufacturer: Millipore, 50cm², 30KD, Cat No.: PXB030A50; Lot No.: C8AA16209-0026
incubator: SFS-160 drug strong-light stability test chamber, and LHS-250SC constant temperature and humidity incubator

The methods used in the following experiments can be as follows:

### Analytical method by SEC-HPLC:

1) A liquid chromatography system and TSK-GEL G3000SWXI, chromatography columns (TOSOH Bioscience, column specification: 7.8 × 300 mm, 5 µm) were used. The column temperature was set to room temperature, the flow rate was 0.5 mL/min, and equilibrium was maintained for 30-60 min until the baseline was stable.
2) The samples were injected, a chromatogram was recorded, and the percentage content of the monomers or aggregates of the test solution was calculated according to the peak area normalization method after integration.

### Analytical method by IEC-HPLC:

### 1) Liquid chromatography system and chromatography column

Instrument: Agilent 1260 Infinity/Waters e2695; chromatography column: Thermo ProPacTM WCX-10 4 × 250 mm, 10 µm; flow rate: 0.8 mL/min; column temperature: 30 °C.

The samples were injected, a chromatogram was recorded, and the percentage contents of the acidic peak, main peak, and basic peak of the test solution were calculated according to the peak area normalization method after integration.

### Analytical method by CE-SDS (NR):

The capillary electrophoresis apparatus was Agilent CE7100. After the samples were injected, a chromatogram was recorded, the data were processed by integration, and the percentage content of the monomer peak was calculated using the area normalization method.

In some embodiments, the recombinant anti-human interleukin-17A fully human monoclonal antibody is secukinumab, such as an antibody in COSENTYX^{®} and biosimilars thereof, which comprises two heavy chains having the same sequence and two light chains having the same sequence, wherein the light chain and heavy chain sequences are respectively:
Light chain sequence: 
EIVLTQSPGTLSLSPGERATLSCRASQSVSSSYLAWYQQKPGQAPRLLIYGASSRATGIPD
RFSGSGSGTDFTLTISRLEPEDFAVYYCQQYGSSPCTFGQGTRLEIKRTVAAPSVFIFPPSD
EQLKSGTASVVCLLNNFYPREAKVQWKVDNALQSGNSQESVTEQDSKDSTYSLSSTLTL
SKADYEKHKVYACEVTHQGLSSPVTKSFNRGEC (SEQ ID NO: 1);
Heavy chain sequence:

EVQLVESGGGLVQPGGSLRLSCAASGFTFSNYWMNWVRQAPGKGLEWVAAINQDGSE
KYYVGSVKGRFTISRDNAKNSLYLQMNSLRVEDTAVYYCVRDYYDILTDYYIHYWYFD
LWGRGTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKRVEPKSCDKTHT
CPPCPAPELLGGPSVFLFPPKPKDTLMISRTPEVTCVVVDVSHEDPEVKFNWYVDGVEVH
NAKTKPREEQYNSTYRVVSVLTVLHQDWLNGKEYKCKVSNKALPAPIEKTISKAKGQPR
EPQVYTLPPSREEMTKNQVSLTCLVKGFYPSDIAVEWESNGQPENNYKTTPPVLDSDGSF
FLYSKLTVDKSRWQQGNVFSCSVMHEALHNHYTQKSLSLSPGK (SEQ ID NO: 2).

Secukinumab used in the examples was expressed by CHO cells, which was prepared after cell culture, separation, and high purification. The CHO cells, antibody expression methods, plasmid construction, purification methods, and the like were purchased and operated according to a conventional manner.

The antibodies used in the following examples were all purified secukinumab.

### Example 1: pH Screening Experiment

Seven citrate buffers at 20 mM with pH values of 5.4, 5.6, 5.8, 6.0, 6.2, 6.4, and 6.6 were prepared, respectively. 20 mM citrate buffer (4.2 g of citric acid monohydrate was weighed and dissolved in 1 L of water) and 20 mM citrate buffer (5.89 g of sodium citrate dihydrate was weighed and dissolved in 1 L of water) were prepared, respectively, and the above two buffers were adjusted with each other into 20 mM citrate buffers with a pH of 5.3 and a pH of 6.6. The secukinumab samples were exchanged into buffers with a pH of 5.3 and a pH of 6.6, respectively. Then the samples with two pH values were adjusted with each other, and the samples at the same concentration with other pH values could be obtained. Finally, antibody formulations at an antibody concentration of 150 mg/mL with pH values of 5.4, 5.6, 5.8, 6.0, 6.2, 6.4, and 6.6 were obtained.

The above formulations were placed in a thermostat incubator at 40 °C, and sampling was performed at week 1, week 2, week 3, and week 4 to investigate their stability.

**Table 1: Effect of buffers with different pH values on antibodies in high-temperature experiment**

| Test item | Group | Day 0 | Week 1 | Week 2 | Week 3 | Week 4 |
|---|---|---|---|---|---|---|
| SEC-aggregates (%) | pH 5.4 | 1.99 | 3.71 | 4.56 | 5.55 | 6.42 |
| | pH 5.6 | 1.85 | 3.55 | 4.3 | 5.17 | 5.8 |
| | pH 5.8 | 1.81 | 3.41 | 3.98 | 4.65 | 5.26 |
| | pH 6.0 | 1.74 | 3.38 | 3.87 | 4.54 | 5.04 |
| | pH 6.2 | 1.66 | 3.34 | 3.75 | 4.32 | 5.07 |
| | pH 6.4 | 1.62 | 3.35 | 3.77 | 4.28 | 4.77 |
| | pH 6.6 | 1.6 | 3.35 | 3.68 | 4.23 | 4.76 |
| SEC-monomer (%) | pH 5.4 | 98.01 | 96.09 | 95.07 | 92.45 | 91.49 |
| | pH 5.6 | 98.15 | 96.27 | 95.37 | 93.01 | 92.34 |
| | pH 5.8 | 98.19 | 96.43 | 95.7 | 93.74 | 93.07 |
| | pH 6.0 | 98.26 | 96.47 | 95.83 | 93.89 | 93.38 |
| | pH 6.2 | 98.34 | 96.52 | 95.98 | 94.16 | 93.37 |
| | pH 6.4 | 98.38 | 96.49 | 95.93 | 94.22 | 93.72 |
| | pH 6.6 | 98.4 | 96.5 | 96.04 | 94.26 | 93.72 |
| IEC-main peak (%) | pH 5.4 | 82.28 | 69.19 | 57.91 | 53.82 | 45.17 |
| | pH 5.6 | 82.03 | 70.84 | 61.27 | 56.72 | 48.64 |
| | pH 5.8 | 82.17 | 73.09 | 64.29 | 60.25 | 52.87 |
| | pH 6.0 | 82.01 | 74.05 | 66.2 | 62.11 | 55.52 |
| | pH 6.2 | 82.41 | 75.56 | 68.65 | 65.46 | 58.22 |
| | pH 6.4 | 82.39 | 76.36 | 69.49 | 66.58 | 60.58 |
| | pH 6.6 | 82.53 | 76.5 | 70.08 | 66.68 | 60.67 |
| CE-SDS (NR)-main peak (%) | pH 5.4 | 98.25 | 96.59 | 95.94 | 95.39 | 94.01 |
| | pH 5.6 | 98.35 | 96.58 | 96.31 | 95.63 | 95.24 |
| | pH 5.8 | 98.45 | 96.68 | 96 | 95.04 | 94.82 |
| | pH 6.0 | 98.16 | 96.65 | 96.48 | 95.65 | 94.65 |
| | pH 6.2 | 98.07 | 96.08 | 96.45 | 96.02 | 95.07 |
| | pH 6.4 | 98.15 | 96.03 | 96.17 | 95.46 | 95.38 |
| | pH 6.6 | 98.13 | 96.42 | 96.49 | 95.81 | 94.48 |

As can be seen from the data in Table 1, the anti-IL-17 antibody has a certain stability in the range of pH 5.4-6.6, and overall, the stability is better as the pH increases. As evaluated by two indicators of SEC-monomer and IEC-main peak (%), the anti-IL-17 antibody has good stability in the range of pH 5.8-6.6, such as pH 5.8, pH 6.0, pH 6.2, pH 6.4, and pH 6.6.

### Example 2: Buffer System Screening

Buffers with different components were prepared and the effect of the different buffers on protein stability was investigated under identical conditions. The components and preparation method of each buffer are shown in Table 2. The antibody prepared by the culture was subjected to ultrafiltration using the buffers to obtain formulations shown in Table 3 for this example.

**Table 2: Buffer preparation**

| Buffer No. | Buffer name | Preparation method (g/L) | pH |
|---|---|---|---|
| 1 | 20 mM PB-CB | 2.10 g of citric acid, 1.4196 g of disodium hydrogen phosphate, then adjusting the pH with 5 M NaOH | 6.40 |
| 2 | 20 mM CB | 4.2 g of citric acid then adjusting the pH with 5 M NaOH | 6.39 |
| 3 | 20 mM PB | 3.04 g of sodium dihydrogen phosphate, then adjusting the pH with 5 M NaOH | 6.50 |

**Table 3: Prepared formulation**

| Formulation No. | PB-CB | CB | PB |
|---|---|---|---|
| Buffer | 20 mM PB-CB | 20 mM CB | 20 mM CB |
| Anti-IL-17 antibody concentration (mg/mL) | 8 | 8 | 8 |
| Volume (mL) | 10 | 10 | 10 |

Each formulation was placed in a thermostat incubator at 40 °C for 4 weeks, and the aggregates and fragment changes of each group of samples were investigated.

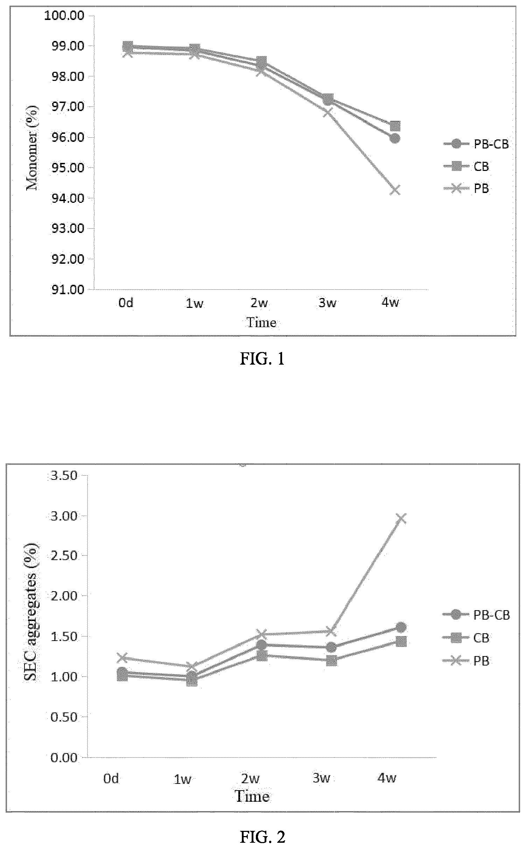

As can be seen from the trend of change in SEC-HPLC monomer purity in FIG. 1, the trends of change of the 3 groups of samples are substantially consistent, but the decrease is significantly different. The CB buffer is significantly better than other groups, followed by PB-CB.

As can be seen from the analysis of the trend of change in SEC-HPLC aggregates over time in FIG. 2, the trends of change in the aggregates contents of the 3 groups of samples are substantially consistent, but the increase is significantly different. The CB buffer is significantly better than other groups, followed by PB-CB.

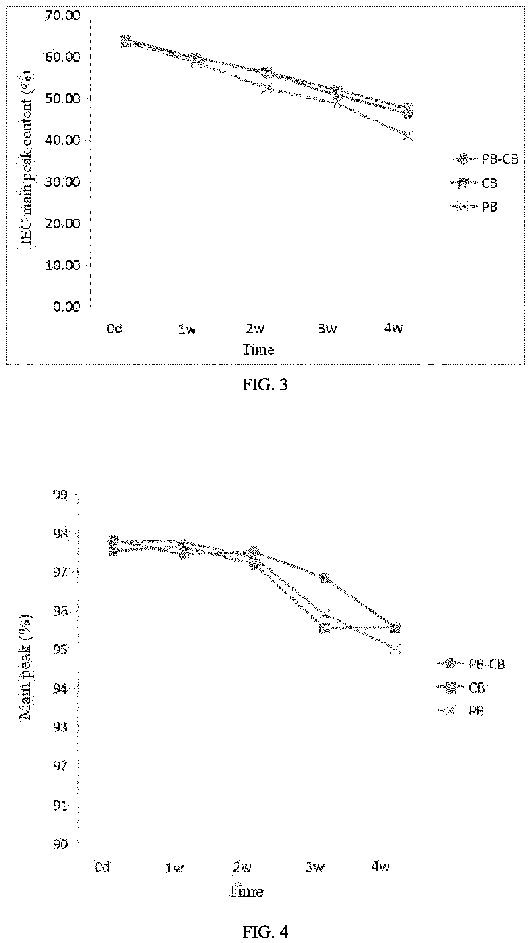

As can be seen from the analysis of the trend of change in IEC-HPLC main peak over time in FIG. 3, the main peak contents of the 3 groups of samples all show a decreasing trend over time, wherein the PB-CB group is equivalent to the CB group, and the decrease is smaller than that of the PB group.

As can be seen from the analysis of the trend of change in CE-SDS (NR) monomer purity over time in FIG. 4, the trends of change of the 3 groups of samples are substantially consistent, and the decrease is not greatly different, indicating that different buffer systems have little effect on the various CE-SDS (NR) indicators of proteins.

Further, the above formulations were subjected to a light test, which were placed at 4000 lx and 25 °C for 14 days, and various indicators were detected.

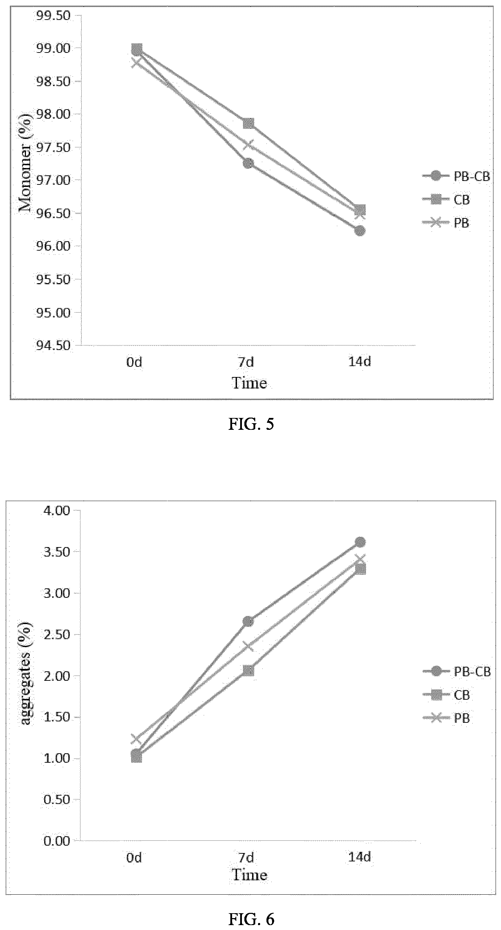

As can be seen from FIG. 5, the monomer contents of the antibody of the 3 groups of samples all decrease to different degrees over time under the light condition, and the CB buffer is the best. As can be seen from the trend of change in the aggregates content in FIG. 6, the increasing trends of the aggregates contents of the 3 groups of samples are substantially consistent, but the increase is different. The aggregates increase in the CB buffer is relatively slowest.

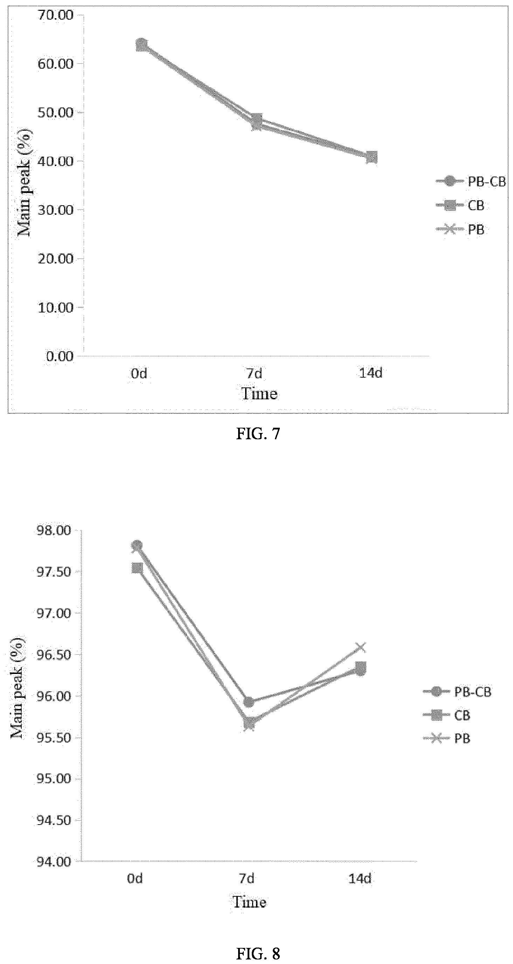

As can be seen from the trend of change in IEC-HPLC main peak over time in FIG. 7, the difference among the groups is not great.

As can be seen from the trend of change in CE-SDS (NR) main peak content in FIG. 8, the main peak contents of the 3 groups of samples all decrease to different degrees under the light condition as compared to Day 0, and the difference among the 3 groups of samples is not great. According to the results of SEC-HPLC, IEC-HPLC, and CE-SDS (NR), it can be seen that the samples have the best stability in the CB buffer under the high temperature of 40 °C and light environment, while the samples have relatively poor stability in the PB or PB-CB buffer. Therefore, the CB buffer can be used as a buffer for the anti-IL-17 antibody formulation.

### Example 3: Stabilizer Screening Experiment

Samples containing different stabilizers were prepared, and the effect of the different stabilizers on antibody stability was investigated under identical conditions (20 mM citrate buffer as the buffer).

The 20 mM citrate buffer was prepared as follows: 21.014 g/4 L of citric acid monohydrate was mixed with 3.68 g/4 L of sodium hydroxide, and the pH value was adjusted to 6.26. The buffer was mixed with other components in each of the following formulations. In each formulation, the anti-IL-17 antibody was at a concentration of 150 mg/mL, and the pH value was 6.26, and the formulations comprised various stabilizers. The specific components of the stabilizer in each formulation were as follows:
KB: blank control, no stabilizer added;
Man: 4% mannitol;
Sor: 4% sorbitol;
Tre: 7.5% trehalose dihydrate;
Suc: 7.5% sucrose;
Arg.HCl: 1% arginine hydrochloride;
NaCl: 1% sodium chloride;
Met: 1% methionine;
EDTA: 1% ethylenediamine tetraacetic acid;
Sor + Met: 4% sorbitol + 1% methionine;
EDTA + Fe²⁺: 1% ethylenediamine tetraacetic acid + 0.5 mM ferrous sulfate heptahydrate; and
EDTA + Fe³⁺: 1% ethylenediamine tetraacetic acid + 0.5 mM ferric chloride hexahydrate.

Each formulation was treated under high temperature and light conditions, and the change of the antibody in each formulation was investigated. The high-temperature treatment condition was as follows: the samples were placed at 40 °C for 4 weeks, and sampling was performed at week 1, week 2, week 3, and week 4 for SEC-HPLC, IEC-HPLC, and CE-SDS (NR) detection. The light treatment condition was as follows: the samples were placed under a light condition of 4000 lx (25 °C) for 10 d (days), and sampling was performed on day 5 and day 10 for SEC-HPLC, IEC-HPLC, and CE-SDS (NR) detection.

Table 4 shows the detection data under the high-temperature treatment. After 4 weeks of the high-temperature experiment, the trend of change in SEC-HPLC monomer content was different. The monomers of the samples containing EDTA decreased rapidly, and in particular, the monomer contents of the formulations containing Fe²⁺ and Fe³⁺ ions decreased most rapidly. The formulations containing Met as well as Met and sorbitol had a relatively small decrease in the monomer content.

As can be seen from the analysis of SEC-HPLC aggregates data, the trends of change of the 12 groups of formulations are substantially consistent, but the increase is different. The aggregates increased more in the formulations containing EDTA, and in particular, the aggregates increased most in the formulations containing Fe²⁺ and Fe³⁺.The formulations containing Met as well as Met and sorbitol had a relatively small increase in the aggregates.

As can be seen from the analysis of IEC-HPLC main peak data, the IEC main peaks of the formulations containing EDTA decrease significantly after the samples were placed under the high-temperature condition, and the formulations containing Fe²⁺ and Fe³⁺ decrease most significantly. Little difference in the stabilizing effects exists among the other various stabilizers. As can be seen from the trend of change in CE-SDS (NR) main peak content, the antibody is relatively stable under the high-temperature condition of 40 °C. However, the antibody has relatively poor stability in the formulations containing EDTA, and in particular, the antibody has the worst stability in the formulations containing Fe²⁺ and Fe³⁺. The main peak contents of the formulations containing other stabilizers have little difference.

**Table 4: Detection results of formulations containing different stabilizers under the high-temperature treatment**

| **Sampling time** | **Sample name** | **SEC aggregat es (%)** | **SEC monomer (%)** | **IEC main peak (%)** | **CE main peak (%)** | **Sampling time** | **Sample name** | **SEC aggregat es (%)** | **SEC monomer (%)** | **IEC main peak (%)** | **CE main peak (%)** |
|---|---|---|---|---|---|---|---|---|---|---|---|
| Day 0 | KB | 1.1 | 98.51 | 63.18 | 97.57 | Week 1 | KB | 2.33 | 97.5 | 57.48 | 96.7 |
| | Man | 1.15 | 98.61 | 63.75 | 97.37 | | Man | 2.24 | 97.59 | 58.12 | 96.74 |
| | Sor | 1.18 | 98.54 | 63.15 | 97.29 | | Sor | 2.22 | 97.66 | 56.64 | 96.69 |
| | Tre | 1.08 | 98.73 | 63.19 | 97.28 | | Tre | 2.13 | 97.72 | 58.63 | 96.55 |
| | Sue | 1.07 | 98.79 | 63.2 | 97.47 | | Sue | 2.13 | 97.7 | 58.1 | 96.58 |
| | Arg.HCl | 0.98 | 98.87 | 63.22 | 97.29 | | Arg.HCl | 1.95 | 97.89 | 57.18 | 96.73 |
| | NaCl | 0.95 | 98.88 | 63.19 | 97.28 | | NaCl | 2.16 | 97.67 | 58.48 | 96.38 |
| | Met | 0.91 | 98.9 | 63.14 | 97.33 | | Met | 1.92 | 97.9 | 58.13 | 96.8 |
| | EDTA | 0.9 | 98.89 | 63.22 | 96.61 | | EDTA | 2.21 | 97.56 | 57.46 | 96.51 |
| | Sor + Met | 0.87 | 98.97 | 63.17 | 96.42 | | Sor + Met | 1.77 | 98.03 | 56.48 | 96.63 |
| | EDTA + Fe²⁺ | 1.03 | 98.8 | 63.08 | 96.79 | | EDTA + Fe²⁺ | 3.89 | 95.78 | 52.72 | 94.81 |
| | EDTA + Fe³⁺ | 1.37 | 98.4 | 62.66 | 96.49 | | EDTA + Fe³⁺ | 5.05 | 94.53 | 51.05 | 93.92 |
| Week 2 | KB | 2.87 | 96.9 | 54.6 | 95.16 | Week 3 | KB | 3.94 | 93.94 | 53.52 | 95.94 |
| | Man | 2.7 | 97.06 | 55 | 95.38 | | Man | 3.52 | 94.54 | 53.84 | 95.29 |
| | Sor | 2.69 | 97.08 | 54.73 | 95.22 | | Sor | 2.68 | 95.86 | 54.45 | 95.82 |
| | Tre | 2.6 | 97.17 | 54.93 | 94.7 | | Tre | 3.23 | 95.05 | 53.53 | 95.67 |
| | Sue | 2.54 | 97.23 | 54.83 | 95.51 | | Suc | 3.14 | 95.11 | 54.08 | 95.77 |
| | Arg.HCl | 2.34 | 97.42 | 55.12 | 95.6 | | Arg.HCl | 2.85 | 95.46 | 53.45 | 95.72 |
| | NaCl | 2.36 | 97.45 | 55.44 | 95.51 | | NaCl | 3.26 | 95.05 | 54.18 | 95.64 |
| | Met | 2.37 | 97.38 | 54.34 | 95.77 | | Met | 2.86 | 95.45 | 52.44 | 95.33 |
| | EDTA | 2.92 | 96.8 | 52.87 | 95.08 | | EDTA | 3.57 | 94.58 | 49.63 | 95.27 |
| | Sor + Met | 2.17 | 97.56 | 54.15 | 95.37 | | Sor + Met | 2.55 | 95.67 | 52.04 | 95.52 |
| | EDTA + Fe²⁺ | 6.37 | 91.02 | 45.64 | 91.91 | | EDTA + Fe²⁺ | 7.5 | 89.54 | 42.93 | 90.97 |
| | EDTA + Fe³⁺ | 7.7 | 89.7 | 44.06 | 91.23 | | EDTA + Fe³⁺ | 8.38 | 88.67 | 42.43 | 91.3 |
| Week 4 | KB | 4.55 | 93.46 | 49.37 | 94.63 | | | | | | |
| | Man | 3.67 | 94.41 | 50.43 | 95.11 | | | | | | |
| | Sor | 3.54 | 94.66 | 50.37 | 94.53 | | | | | | |
| | Tre | 3.44 | 94.69 | 50.44 | 94.78 | | | | | | |
| | Suc | 3.41 | 94.75 | 50.5 | 94.79 | | | | | | |
| | Arg.HCl | 3.09 | 95.02 | 49.2 | 95.04 | | | | | | |
| | NaCl | 3.74 | 94.39 | 50.2 | 94.76 | | | | | | |
| | Met | 3.24 | 94.82 | 50.41 | 94.78 | | | | | | |
| | EDTA | 4.29 | 93.61 | 45.35 | 94.37 | | | | | | |
| | Sor + Met | 2.98 | 94.97 | 48.03 | 94.72 | | | | | | |
| | EDTA + Fe²⁺ | 10.4 | 85.96 | 35.65 | 90.03 | | | | | | |
| | EDTA + Fe³⁺ | 11.21 | 85.37 | 35.01 | 89.51 | | | | | | |

**Table 5: Detection results of formulations containing different stabilizers in the light experiment**

| **Sampling time** | **Sample name** | **SEC aggregat es (%)** | **SEC monomer (%)** | **IEC main peak (%)** | **CE main peak (%)** | **Sampling time** | **Sample name** | **SEC aggregat es (%)** | **SEC monomer (%)** | **IEC main peak (%)** | **CE main peak (%)** |
|---|---|---|---|---|---|---|---|---|---|---|---|
| Day 0 | KB | 1.1 | 98.51 | 63.18 | 97.57 | Day 5 | KB | 8.41 | 91.49 | 41.85 | 93.3 |
| | Man | 1.15 | 98.61 | 63.75 | 97.37 | | Man | 6.97 | 92.93 | 43.59 | 94.25 |
| | Sor | 1.18 | 98.54 | 63.15 | 97.29 | | Sor | 7.45 | 92.44 | 42.59 | 93.92 |
| | Tre | 1.08 | 98.73 | 63.19 | 97.28 | | Tre | 6.57 | 93.33 | 44.39 | 94.25 |
| | Sue | 1.07 | 98.79 | 63.2 | 97.47 | | Sue | 7.07 | 92.84 | 43.58 | 93.86 |
| | Arg.HCl | 0.98 | 98.87 | 63.22 | 97.29 | | Arg.HCl | 5 | 94.9 | 46.93 | 95 |
| | NaCl | 0.95 | 98.88 | 63.19 | 97.28 | | NaCl | 5.9 | 94.01 | 46.23 | 94.53 |
| | Met | 0.91 | 98.9 | 63.14 | 97.33 | | Met | 4.05 | 95.85 | 50.35 | 95.63 |
| | EDTA | 0.9 | 98.89 | 63.22 | 96.61 | | EDTA | 4.34 | 95.55 | 44.73 | 95.51 |
| | Sor + Met | 0.87 | 98.97 | 63.17 | 96.42 | | Sor + Met | 3.21 | 96.7 | 53.46 | 96.22 |
| | EDTA + Fe²⁺ | 1.03 | 98.8 | 63.08 | 96.79 | | EDTA + Fe²⁺ | 5.43 | 94.42 | 46.21 | 94.58 |
| | EDTA + Fe³⁺ | 1.37 | 98.4 | 62.66 | 96.49 | | EDTA + Fe³⁺ | 6 | 93.86 | 44.79 | 94.13 |
| Day 10 | KB | 11.54 | 88.34 | 32.14 | 90.96 | | | | | | |
| | Man | 9.82 | 90.07 | 33.89 | 91.68 | | | | | | |
| | Sor | 9.29 | 90.6 | 36.96 | 92.01 | | | | | | |
| | Tre | 11.15 | 88.73 | 32.89 | 91.07 | | | | | | |
| | Sue | 10.02 | 89.87 | 33.37 | 91.61 | | | | | | |
| | Arg.HCl | 8.85 | 91.03 | 31.49 | 92.35 | | | | | | |
| | NaCl | 8.72 | 91.17 | 35.55 | 92.51 | | | | | | |
| | Met | 4.97 | 94.92 | 46.34 | 94.62 | | | | | | |
| | EDTA | 7.87 | 91.99 | 29.03 | 92.48 | | | | | | |
| | Sor + Met | 4.29 | 95.61 | 47.03 | 95.22 | | | | | | |
| | EDTA + Fe²⁺ | 10.02 | 89.77 | 34.88 | 91.09 | | | | | | |
| | EDTA + Fe³⁺ | 10.52 | 89.26 | 36.52 | 89.92 | | | | | | |

Table 5 shows the experimental results under the light condition. As can be seen from the trend of change in SEC-HPLC monomer content, the monomer contents of the 12 groups of samples all decrease slightly over time. As can be seen from the decrease, the formulations containing Met as well as Met and Sor have the highest monomer content after 10 d (days) of light.

As can be seen from the analysis of SEC-HPLC aggregates data, an increase in aggregates exists among all the 12 groups of samples, but the increase is slightly different: the aggregates increase significantly in the KB group, Tre group, Suc group, EDTA + Fe²⁺ group, and EDTA + Fe³⁺ group, while the aggregates increase less in the formulations containing Met as well as Met and sorbitol.

As can be seen from the analysis of IEC-HPLC main peak data, after 10 days of light, the formulations containing Sor + Met and Met have the highest main peak value, that is, the formulations are the most stable.

As can be seen from the trend of change in CE-SDS (NR) main peak content, the target protein shows a decreasing trend under the light condition. In particular, similarly, the formulations containing Sor + Met and Met have the highest main peak value, that is, the formulations are the most stable.

According to the experimental results under high-temperature and light conditions, it can be seen that Met and Met + Sor perform very well in SEC-HPLC, IEC-HPLC, and CE-SDS (NR), and the combination of sorbitol and methionine has the best stability. Methionine or a combination of sorbitol and methionine can be used as effective stabilizers.

### Example 4: Selection of Methionine Content

Samples containing different contents of methionine (Met) were prepared, and the effect of methionine content on antibody stability was investigated under identical conditions. The buffer for the samples was 20 mM histidine hydrochloride buffer: 2.17 g/L of histidine (His) and 1.257 g/L of His·HCl·H₂O (histidine hydrochloride (Merck, Cat. No.: 104354)) were taken and the pH was adjusted to 6.3. The buffer exchange by ultrafiltration was performed on the prepared anti-IL-17 antibody, and the formulations used in this example were prepared. The anti-IL-17 antibody was at a concentration of 20 mg/mL, and the pH value was 6.3 in each formulation. The components contained in each formulation are shown in Table 6. High-temperature and light experiments were performed on each formulation, and the change of the antibody in each formulation was investigated. The high-temperature treatment condition was as follows: the samples were placed at 40 °C for 4 weeks, and sampling was performed at week 1, week 2, week 3, and week 4 for SEC-HPLC and IEC-HPLC detection. The light treatment condition was as follows: the samples were placed under a light condition of 4000 lx (25 °C) for 10 days, and sampling was performed on day 5 and day 10 for SEC-HPLC and IEC-HPLC detection.

**Table 6: Formulations containing different concentrations of methionine**

| Formulation No. | Formulation |
|---|---|
| 1 | Histidine hydrochloride buffer + antibody + 1% Met |
| 2 | Histidine hydrochloride buffer + antibody + 0.5% Met |
| 3 | Histidine hydrochloride buffer + antibody + 0.25% Met |
| 4 | Histidine hydrochloride buffer + antibody + 0.15% Met |
| 5 | Histidine hydrochloride buffer + antibody + 0.075% Met |
| 6 | Histidine hydrochloride buffer + antibody + 0.05% Met |
| 7 | Histidine hydrochloride buffer + antibody + 0.075% Met + 7.5% HZT (trehalose dihydrate) |
| 8 | Histidine hydrochloride buffer + antibody |

Table 7 shows the change in quality of samples with different contents of methionine under the high-temperature condition (40 °C).

As can be seen from the trend of change in SEC-HPLC monomer content, the monomers of the samples with different concentrations of methionine all show a decreasing trend after the samples were placed at the high temperature of 40 °C, and the difference among the groups is not great. As can be seen from the analysis of SEC-HPLC aggregates data, the trends of change of the 8 groups of samples are substantially consistent, but the increase is slightly different, and the overall difference is not significant.

As can be seen from the analysis of IEC-HPLC main peak data, the main peaks of the samples with different contents of methionine have no significant difference after the samples were placed under the high-temperature condition.

**Table 7: Change in each indicator of different concentrations of Met over time in the high-temperature experiment**

| Test item | Time | Formulation No. | | | | | | | |
|---|---|---|---|---|---|---|---|---|---|
| | | 1 | 2 | 3 | 4 | 5 | 6 | 7 | 8 |
| SEC aggregates (%) | Day 0 | 0.91 | 0.88 | 0.92 | 0.86 | 0.89 | 0.93 | 0.96 | 0.83 |
| | Week 1 | 0.72 | 0.7 | 0.71 | 0.72 | 0.76 | 0.77 | 0.69 | 0.85 |
| | Week 2 | 0.75 | 0.81 | 0.81 | 0.84 | 0.75 | 0.9 | 0.75 | 0.91 |
| | Week 3 | 0.84 | 0.87 | 0.92 | 0.91 | 0.98 | 1 | 0.92 | 1.04 |
| | Week 4 | 1.2 | 1.09 | 1.08 | 1.04 | 1.11 | 1.07 | 0.92 | 1.21 |
| SEC monomer (%) | Day 0 | 99.09 | 99.12 | 99.08 | 99.14 | 99.11 | 99.07 | 99.04 | 99.17 |
| | Week 1 | 99.02 | 99.05 | 99.04 | 99.02 | 99 | 98.99 | 99.06 | 98.88 |
| | Week 2 | 98.87 | 98.71 | 98.79 | 98.79 | 98.89 | 98.73 | 98.9 | 98.69 |
| | Week 3 | 97.28 | 97.3 | 97.21 | 97.3 | 97.15 | 97.25 | 96.98 | 96.93 |
| | Week 4 | 96.3 | 96.43 | 96.49 | 96.59 | 96.15 | 96.54 | 96.7 | 96.27 |
| IEC main peak (%) | Day 0 | 62.83 | 62.71 | 62.74 | 62.63 | 62.62 | 62.7 | 62.53 | 62.42 |
| | Week 1 | 60.79 | 60.02 | 60.44 | 60.09 | 60 | 59.61 | 60.47 | 59.21 |
| | Week 2 | 55.49 | 55.21 | 55.49 | 55.37 | 55.51 | 55.38 | 55.18 | 55.51 |
| | Week 3 | 51.93 | 52 | 52.09 | 51.79 | 51.84 | 51.86 | 51.23 | 51.35 |
| | Week 4 | 48.97 | 48.64 | 48.86 | 49.42 | 48.91 | 48.67 | 48.71 | 47.44 |

Table 8 shows the detection results of the samples with different contents of methionine added under the light condition. As can be seen from the trend of change in SEC-HPLC monomer content, the monomer contents of the 8 groups of samples all decrease over time. Formulations 1, 2, 3, and 4 (0.15%-1% Met) are preferred from the standpoint of the decrease and the comparison with the control formulation (formulation 7).

As can be seen from the analysis of IEC-HPLC main peak data, the main peak contents of the 8 groups of samples all show a decreasing trend along with the prolonging of the light time. As can be seen from the analysis, formulations 1, 2, 3, and 4 (0.15%-1% Met) are preferred compared to the control formulation (formulation 7) after the samples were placed under the light condition.

**Table 8: Experimental results of formulations with different concentrations of Met in the light experiment**

| Test item | Time | Formulation No. | | | | | | | |
|---|---|---|---|---|---|---|---|---|---|
| | | 1 | 2 | 3 | 4 | 5 | 6 | 7 | 8 |
| SEC aggregates (%) | Day 0 | 0.91 | 0.88 | 0.92 | 0.86 | 0.89 | 0.93 | 0.96 | 0.83 |
| | Day 7 | 0.83 | 0.88 | 0.74 | 1.12 | 1.22 | 1.18 | 0.82 | 1.43 |
| | Day 14 | 1.04 | 1.11 | 1.1 | 1.28 | 1.99 | 1.58 | 1.22 | 2.45 |
| SEC monomer (%) | Day 0 | 99.09 | 99.12 | 99.08 | 99.14 | 99.11 | 99.07 | 99.04 | 99.17 |
| | Day 7 | 98.95 | 98.99 | 99.13 | 98.75 | 98.6 | 98.63 | 99.03 | 98.37 |
| | Day 14 | 98.82 | 98.8 | 98.77 | 98.59 | 97.81 | 98.26 | 98.63 | 97.29 |
| IEC main peak (%) | Day 0 | 62.83 | 62.71 | 62.74 | 62.63 | 62.62 | 62.7 | 62.53 | 62.42 |
| | Day 7 | 56.63 | 54.35 | 51.03 | 51.39 | 51.49 | 50.24 | 51.51 | 46.8 |
| | Day 14 | 50.99 | 44.26 | 41.49 | 40.15 | 33.02 | 37.38 | 38.72 | 31.67 |

According to the experimental results under high-temperature and light conditions, 0.15%-1% Met is preferred as a stabilizer compared to the control formulation (formulation 7).

### Example 5: Surfactant Screening Experiment

Samples with different surfactants and samples with different concentrations of the surfactant were prepared and investigated for their stability by shaking test (200 rpm, room temperature). Formulations containing different types and concentrations of surfactants were prepared. In each formulation, the anti-IL-17 antibody was at a concentration of 150 mg/mL, the pH value was 6.26, and the buffer was 20 mM citrate buffer. The components and content of each formulation are shown in Table 9, where TW20 is Tween 20, and TW80 is Tween 80.

The preparation method of each formulation was as follows: 20 mM citrate buffer (4.2 mg of citric acid monohydrate was weighed based on 1 mL of the buffer, and the pH was adjusted to 6.3 with 5 M NaOH) was first prepared, the anti-IL-17 antibody was exchanged by ultrafiltration into 20 mM citrate buffer with a pH of 6.3, and the final antibody concentration was 150 mg/mL. 0.01%-0.04% surfactants (TW20 or TW80) or no surfactants were added to the above antibody solutions, respectively, and the mixed solutions were mixed uniformly.

**Table 9: Formulations containing different types and concentrations of surfactants**

| Formulation No. | Components |
|---|---|
| KB | Anti-IL-17 antibody (no surfactant) + buffer |
| 0.01% TW20 | 0.01% TW20 + anti-IL-17 antibody + buffer |
| 0.02% TW20 | 0.02% TW20 + anti-IL-17 antibody + buffer |
| 0.04% TW20 | 0.04% TW20 + anti-IL-17 antibody + buffer |
| 0.01% TW80 | 0.01% TW80 + anti-IL-17 antibody + buffer |
| 0.02% TW80 | 0.02% TW80 + anti-IL-17 antibody + buffer |
| 0.04% TW80 | 0.04% TW80 + anti-IL-17 antibody + buffer |

After shaking, the turbidity change of each formulation was investigated. The results are shown in Table 10.

**Table 10: Turbidity (OD340) changes of sample solutions containing different types and contents of surfactants after shaking**

| Formulation No. | 0 h | 2 h | 4h | 8 h | 24 h | 48 h | 72 h |
|---|---|---|---|---|---|---|---|
| KB | 0.457 | 0.671 | 0.613 | 0.731 | 0.812 | 0.882 | 1.636 |
| 0.01% TW20 | 0.427 | 0.451 | 0.427 | 0.468 | 0.469 | 0.454 | 0.512 |
| 0.02% TW20 | 0.415 | 0.436 | 0.468 | 0.4 | 0.422 | 0.442 | 0.457 |
| 0.04% TW20 | 0.415 | 0.416 | 0.406 | 0.411 | 0.412 | 0.423 | 0.427 |
| 0.01% TW80 | 0.412 | 0.416 | 0.408 | 0.41 | 0.414 | 0.43 | 0.441 |
| 0.02% TW80 | 0.418 | 0.408 | 0.409 | 0.403 | 0.416 | 0.421 | 0.436 |
| 0.04% TW80 | 0.415 | 0.409 | 0.41 | 0.414 | 0.415 | 0.422 | 0.43 |

As can be seen from the trend of change in turbidity of the solutions in Table 10, after being placed for 2 hours (h) under the shaking condition, the blank sample began to be significantly turbid, while the samples with the surfactants added still remained clear after shaking for 72 h, indicating that the surfactants have a certain protection effect on the samples under the shaking condition, can prevent protein aggregation from being turbid, and plays a role in solubilization. In order to further analyze the samples containing the surfactants, the turbidity values of the samples containing Tween 80 are significantly lower than those of the samples containing Tween 20 at different time points when the concentration is 0.01-0.02%. No significant difference in turbidity exists among different concentrations of Tween 80; the solubilization effect of Tween 80 is better than that of Tween 20, so Tween 80 is selected as a surfactant for the anti-IL-17 antibody. Further, the formulations in Table 9 were placed at -20 °C and 25 °C for 12 hours, respectively, to investigate their stability. Specifically, placing at -20 °C and 25 °C for 12 hours (h) was one repeated freeze-thaw cycle, and the cycle was repeated 5 times. The sampling time points were at 0 cycles, 3 cycles, and 5 cycles, and the sub-visible particles were detected. The results are shown in Table 11.

**Table 11: Particle changes of sample solutions containing different types and contents of surfactants after 5 repeated freeze-thaw cycles**

| Sample name | 0 cycles | | 3 cycles | | 5 cycles | |
|---|---|---|---|---|---|---|
| | ≥ 10 µm | ≥ 25 µm | ≥ 10 µm | ≥ 25 µm | ≥ 10 µm | ≥ 25 µm |
| KB | 17.92 | 0.83 | 179.17 | 2.92 | 310.42 | 7.5 |
| 0.01% TW20 | 32.5 | 2.08 | 52.92 | 2.5 | 62.92 | 4.58 |
| 0.02% TW20 | 88.75 | 8.75 | 41.67 | 0.42 | 80 | 5.42 |
| 0.04% TW20 | 15.83 | 0.42 | 101.67 | 6.25 | 122.5 | 5 |
| 0.01% TW80 | 102.5 | 3.33 | 62.08 | 0.83 | 77.92 | 2.08 |
| 0.02% TW80 | 21.67 | 1.25 | 64.17 | 2.08 | 79.58 | 1.67 |
| 0.04% TW80 | 22.92 | 1.67 | 108.33 | 2.92 | 86.67 | 0.42 |

After 5 repeated freeze-thaw cycles, the number of particles in the samples with the surfactants added was significantly lower than that in the samples without the surfactants, indicating that the surfactants can effectively prevent protein aggregation during the repeated freeze-thaw process. Tween 80 has a better ability to reduce particles among different types of surfactants. In order to ensure that the Tween content would be sufficient to provide protection and not too high, 0.02% Tween 80 was selected as a surfactant for the anti-IL-17 antibody, according to the experimental data above.

### Example 6: Formulation Experiment

According to the results of all the above experiments, the preferred formulation of the present invention comprises: 150 mg/mL anti-IL-17 antibody, 20 mM citrate buffer, 3% sorbitol, 0.4% methionine, and 0.02% Tween 80, with a pH value of 5.8-6.4, preferably 6.1.

The preparation method of the formulation could be as follows: based on 1 mL of the formulation, 150 mg of anti-IL-17 antibody, 0.59 mg of citric acid monohydrate, 5.06 mg of sodium citrate dihydrate, 30 mg of sorbitol, 4 mg of methionine, and 0.2 mg of Tween 80 were taken, the volume was brought to 1 mL with water, and the above substances were dissolved and mixed uniformly with a pH of about 6.1.

The preparation method of the formulation could be as follows:

### 1) Buffer preparation

Ultrafiltration buffer (5 L): 2.94 g of citric acid monohydrate, 25.30 g of sodium citrate dihydrate, and 20.00 g of methionine were weighed and dissolved with water for injection, and the volume was brought to 5 L, with a pH of 6.1 ± 0.3.

10% (w/v) polysorbate 80 stock solution (10 mL): 1.00 g of polysorbate 80 was weighed and dissolved with water for injection, and the volume was brought to 10 mL.

16× formulation buffer stock solution (50 mL): 0.0295 g of citric acid monohydrate, 0.2530 g of sodium citrate dihydrate, 0.20 g of methionine, and 24.00 g of sorbitol were weighed and dissolved with water for injection, 1.6 mL of 10% (w/v) polysorbate 80 stock solution was then added, and the volume was brought to 50 mL with water for injection. The pH of the solution was 6.1 ± 0.3.

### 2) Buffer exchange by ultrafiltration and diluting preparation

The buffer exchange by ultrafiltration was performed on the purified anti-IL-17 antibody using an ultrafiltration buffer, and the solution was concentrated to 160 mg/mL after the buffer exchange was completed, which was UF.

The 16× formulation buffer stock solution was added at a ratio of UF:16× formulation buffer stock solution = 15:1 to obtain the target formulation.

### Stability verification

The anti-IL-17 antibody formulation of the above formulation (the formulation in Example 6 with a pH of 6.1) was prepared and numbered 01; a control formulation (the formulation was 150 mg/mL of anti-IL-17 antibody, L-His/His-HCl (3.103 mg/mL; 0.71 mg/mL of histidine and 2.393 mg/mL of histidine hydrochloride (Merck, Cat. No.: 104354)), 0.746 mg/mL of methionine, 0.2 mg/mL of Tween 80, and 75.67 mg/mL of trehalose dihydrate, with a pH of 5.8) was prepared and numbered 02. The above 2 formulations were placed at 25 °C for 5 months, and their stability was investigated. The SEC-HPLC results are shown in Table 12.

**Table 12: Changes in SEC-HPLC monomer (%) of anti-IL-17 antibody formulation at room temperature 25 °C)**

| No. | Day 0 | Month 1 | Month 2 | Month 3 | Month 5 |
|---|---|---|---|---|---|
| 01 | 98.53 | 98.03 | 96.99 | 96.65 | 95.75 |
| 02 | 98.42 | 97.69 | 96.83 | 96.36 | 95.52 |

As can be seen from the results, the SEC-HPLC monomer content of the antibody is relatively high in the anti-IL-17 antibody formulation of the present invention. The anti-IL-17 antibody formulation of the present invention has good stability under the condition of room temperature.

## Claims

1. An antibody formulation, comprising an anti-IL-17 antibody, a buffer, a stabilizer, and a surfactant, wherein the stabilizer is selected from methionine and a combination of methionine and a sugar alcohol, and the antibody formulation has a pH value of 5.4-6.6.

2. The antibody formulation according to claim 1, wherein the antibody formulation has a pH value of 5.8-6.6.

3. The antibody formulation according to claim 1, wherein the antibody formulation has a pH value of 5.8-6.4.

4. The antibody formulation according to claim 1, wherein the antibody formulation has a pH value of 6.1.

5. The antibody formulation according to any one of claims 1-4, wherein the methionine is at a concentration of 0.15%-1%.

6. The antibody formulation according to claim 5, wherein the methionine is at a concentration of 0.4% or 1%.

7. The antibody formulation according to any one of claims 1-6, wherein the sugar alcohol is at a concentration of 3%-5%.

8. The antibody formulation according to claim 7, wherein the sugar alcohol is at a concentration of 3% or 4%.

9. The antibody formulation according to any one of claims 1-8, wherein the buffer is a citrate buffer and/or a phosphate buffer.

10. The antibody formulation according to claim 9, wherein the buffer is a citrate buffer.

11. The antibody formulation according to any one of claims 1-10, wherein the buffer is at a concentration of 10-30 mM.

12. The antibody formulation according to claim 11, wherein the buffer is at a concentration of 20 mM.

13. The antibody formulation according to any one of claims 1-12, wherein the surfactant is Tween 80 or Tween 20.

14. The antibody formulation according to claim 13, wherein the surfactant is Tween 80.

15. The antibody formulation according to any one of claims 1-14, wherein the surfactant is at a concentration of 0.01%-0.04%.

16. The antibody formulation according to claim 15, wherein the surfactant is at a concentration of 0.02%.

17. The antibody formulation according to any one of claims 1-16, wherein the anti-IL-17 antibody is at a concentration of 100-300 mg/mL.

18. The antibody formulation according to claim 17, wherein the anti-IL-17 antibody is at a concentration of 150 mg/mL.

19. The antibody formulation according to any one of claims 1-18, wherein the anti-IL-17 antibody has a light chain sequence set forth in SEQ ID NO: 1, and a heavy chain sequence set forth in SEQ ID NO: 2.

20. The antibody formulation according to any one of claims 1-19, wherein the anti-IL-17 antibody is a monoclonal antibody expressed by a CHO cell.

21. The antibody formulation according to any one of claims 1-20, wherein the antibody formulation comprises 150 mg/mL anti-IL-17 antibody, 20 mM citrate buffer, 3% sugar alcohol, 0.4% methionine, and 0.02% Tween 80, with a pH value of 5.8-6.4.

22. The antibody formulation according to any one of claims 1-21, wherein the sugar alcohol is sorbitol.

23. A method for preparing the antibody formulation according to any one of claims 1-22, comprising taking a specified amount of an anti-IL-17 antibody, a buffer, a stabilizer, and a surfactant, adding water for dissolving, mixing the above substances uniformly, and adjusting the volume to a specified volume.

24. Use of the antibody formulation according to any one of claims 1-22 in the preparation of a product for treating an autoimmune disease.

25. The use according to claim 24, wherein the autoimmune disease is rheumatoid arthritis, psoriatic arthritis, diabetes, asthma, chronic plaque psoriasis, or multiple sclerosis.
